# EUROPEAN PATENT SPECIFICATION

(11) **EP 3 308 819 B1**
(45) Date of publication and mention of the grant of the patent: **11.12.2019**
(21) Application number: 17196487.7
(22) Date of filing: 13.10.2017
(51) Int. Cl.: A61M 16/10, A61M 16/00

(54) **APPARATUS TO IDENTIFY RESPIRATORY ASYNCHRONIES IN AN ASSISTED BREATHING MACHINE**
VORRICHTUNG ZUR IDENTIFIZIERUNG VON ATEMWEGS-ASYNCHRONIE IN EINER VORRICHTUNG ZUR ATMUNGSUNTERSTÜTZUNG
APPAREIL POUR IDENTIFIER DE L'ASYNCHRONIE RESPIRATOIRE DANS UN APPAREIL D'ASSISTANCE RESPIRATOIRE

(30) Priority: 14.10.2016 IT 201600103298
(43) Date of publication of application: 18.04.2018
(73) Proprietor: Università degli Studi di Trieste, 34127 Trieste (IT)
(72) Inventor: Lucangelo, Umberto, 34010 Sgonico (TS) (IT); Fabris, Francesco, 34134 Trieste (TS) (IT); Bortolussi, Luca, 34127 Trieste (TS) (IT); Casagrande, Alberto, 33050 Lestizza (UD) (IT); Borelli, Massimo, 34127 Trieste (TS) (IT); Quintavalle, Francesco, 33010 Pagnacco (UD) (IT)
(74) Representative: Petraz, Gilberto Luigi

(56) References cited:
- EP-A1- 0 671 180
- WO-A1-2016/063172
- GB-A- 2 417 206
- US-A- 6 099 481
- US-A1- 2008 294 060
- US-A1- 2012 204 875
- US-A1- 2016 135 713

## Description

### FIELD OF THE INVENTION

The present invention concerns an apparatus for detecting possible asynchronies that can occur between the conditions of inspiration and expiration set by an assisted breathing machine and the inspiration and expiration activity attempted by a patient partly incapable of breathing independently and associated with said machine.

### BACKGROUND OF THE INVENTION

In intensive care, emergency medicine and/or pulmonology wards, the use of assisted breathing machines is known, which allow to manage the breathing cycle of a patient who is partly or completely unable to breathe independently.

Assisted breathing machines, also called pulmonary ventilators, are normally configured to manage a patient's breathing by providing air to the lungs with a predetermined cadence, divided into an active period, called inspiration phase, and a passive period called the expiration phase. If the patient is subjected to deep sedation, for example in the case of pharmacologically induced muscular paralysis, all respiratory activity is delegated to the pulmonary ventilator that controls the breathing cycle, as the patient is completely passive.

Conversely, in cases where there is only a partial sedation of the patient, or when the patient is to be helped, the respiration is assisted and it is the patient who automatically activates the impulse that regulates the inspiration and expiration phases. This is because the machine is synchronized with the inspiration and expiration stimuli of the patient by means of the control sensors of the pulmonary ventilator, which normally detect volume, pressure and flow of the air transferred.

During partly assisted breathing, pathological or respiratory asynchronies can frequently occur, which happen whenever there is an imperfect synchronization between the inspiration and expiration phases of the patient and the phases of the pulmonary ventilator. For example, a respiratory asynchrony can be characterized by a failed or delayed action of the pulmonary ventilator with respect to the initiation impulse of a respiratory phase.

Respiratory asynchronies can also be very dangerous, and can even cause serious and permanent damage.

One of the main problems of any type of pulmonary ventilator is that such respiratory asynchronies cannot be detected by the machine automatically and in real time without the use of invasive devices. Therefore, it is not possible to intervene in a short time to modify the parameters of the pulmonary ventilator and to synchronize the inspiration and expiration phases induced with those of the patient.

Respiratory asynchronies are always detected after they have occurred, generally by specialists who analyze the data and/or diagram of the temporal trend of volume, pressure, and flow of air delivered and expired by the pulmonary ventilator. Visually, asynchronies appear as oscillatory irregularities in the temporal trend. They are, however, generally not very obvious and therefore difficult to recognize even for highly qualified personnel.

The current status of such machines provides a manual detection and/or regulation that does not guarantee an effective and safe service for patients assisted by pulmonary ventilators, as it depends significantly on the experience of the person who analyzes the data and/or diagrams and on the monitoring frequency by the specialized operators.

A solution is also known that provides to detect the electrical signal that reaches the diaphragm as an impulse from the patient's brain, using a sensor installed on the nasogastric probe normally employed to feed the patient, so as to synchronize the pulmonary ventilator with this signal and create the best possible conditions of assisted breathing.

This solution, however, is at least partly invasive for the patient and can itself cause other problems.

Solutions are known that provide to control the functioning of an assisted breathing machine to monitor a patient's condition, for example from WO-A-2016/063172, US-B-6,099,481, US-A-2008/0294060, US-A-2012/0204875 and GB-A-2,417,206.

Document WO-A-2016/063172 concerns a system configured to detect the presence of secretions in the respiratory tract of a patient during respiratory therapy. The system described in WO-A-2016/063172 comprises detection devices to detect one or more parameters relating to the air delivered and expired at different times during the respiratory therapy.

Document US-B-6,099,481 concerns a method and apparatus to monitor a variety of parameters of a respiratory profile to derive information relating to breathing-related functions and carbon dioxide levels in a patient's blood.

Document US-A-2008/0294060 concerns devices and methods to detect, monitor and/or manage heart or respiratory disorders. The device described in US-A-2008/0294060 is an implantable medical device that comprises a first sensor configured to produce a first signal indicating the variation in volume of the lungs during respiration, a second sensor configured to produce a second signal indicating an intra-pleural pressure and a processor to perform various operations with the volume and pressure signals.

Document US-A-2012/0204875 concerns a method and apparatus for a mechanical ventilation system. The system provides a sensor to measure a characteristic of the air conveyed by the ventilation system and a processor to process the data detected by the sensor and to show in a graph the characteristics measured of the air in a first period and at least one second period, following the first period.

Document GB-A-2.417.206 concerns a method to automatically trace the pressure-volume curves during artificial respiration with a breathing apparatus.

The solutions described in the above documents, however, are not suitable to identify the presence of respiratory asynchronies in a single respiratory act, that is, they are not suitable to monitor the synchronization between the inspiration and expiration phases of the patient and the phases of the pulmonary ventilator inside a single respiratory act.

One purpose of the present invention is to provide an apparatus to control and regulate the parameters of the pulmonary ventilator that allows to detect respiratory asynchronies in a simple and automatic manner, so as to manage the functioning of the pulmonary ventilator in order to eliminate the pathological condition associated with the presence of asynchronies, or at least to make it not dangerous for the patient.

Another purpose of the present invention is to provide an apparatus to control and manage specific parameters of a pulmonary ventilator, which allows to evaluate the intensity and frequency of respiratory asynchronies, so that it can intervene to signal alarms, eliminate the asynchronies, or make them not dangerous for the patient.

Another purpose of the present invention is to associate and/or correlate the detections obtained in order to obtain diagrams that are easy to read and interpret so as to allow to quickly identify any respiratory asynchronies.

Another purpose is to provide a processing system that reads the results of the factors detected, interprets them and conditions the functioning of the machine itself, correlating them to the exact needs of the patient.

The Applicant has devised, tested and embodied the present invention to overcome the shortcomings of the state of the art and to obtain these and other purposes and advantages.

### SUMMARY OF THE INVENTION

The present invention is set forth and characterized in the independent claim, while the dependent claims describe other characteristics of the invention or variants to the main inventive idea.

Embodiments described here concern an apparatus that, on the basis of the information supplied by the assisted breathing machine, allows to monitor the functioning parameters of the pulmonary ventilator so that possible respiratory asynchronies can be identified. It is known that a pulmonary ventilator is provided with a ventilation device configured to deliver a determinate flow of air, at regular intervals and consistent with the needs of a patient, allowing the subsequent outflow thereof.

It is also known that the pulmonary ventilator includes a management unit able to manage the functioning of the ventilation device, in a predefined manner that can be modified by specialists.

It is also known that a pulmonary ventilator has sensors able to detect the parameters of the air delivered/expired, in particular at least the air flow and pressure, said sensors supplying the real information relating to the trend of the air delivered and expired.

In particular, the flow identifies the quantity of air in the unit of time, the pressure defines the pressure present in the air passage tubes during the delivery phase (positive) and the expiration phase (negative), while the volume defines the overall quantity of air delivered and expired.

According to the invention the volume can be also detected according to fractions of the delivery and expiration cycle.

In accordance with one aspect of the present invention, the apparatus is connected to a pulmonary ventilator, and comprises a processing unit able to receive the data detected by the sensors, to select at least the flow and pressure parameters and to process them in order to identify possible respiratory asynchronies inside a complete breathing cycle, comprising a delivery phase and an expiration phase.

In particular, the processing unit can correlate the flow and pressure parameters with respect to each other, and generate a closed curve profile that identifies, on each occasion, a complete delivery and expiration cycle.

By "closed curve" we mean generally a curve that begins and ends substantially at the same point.

According to some embodiments, the apparatus provides to use exclusively the pressure and flow parameters to identify the respiratory asynchronies.

According to the present invention, the processing unit provides to select the pressure and flow parameters and show the respective values in a graph, constructing a main closed pressure-flow curve of at least one complete breathing cycle comprising the phases of delivery and expiration, and one or more secondary closed curves overlapping the main closed curve, each corresponding to a respiratory asynchrony.

The secondary closed curves enclose respective areas of smaller sizes than the area enclosed by the main closed curve.

By the term "overlapping" we mean that the secondary closed curve or curves begin and end at respective points of the main closed curve, and can at least partly intersect the latter.

The main pressure-flow closed curve can be analyzed by specialists, who can identify the presence of a consistent respiratory asynchrony in the presence of at least a secondary pressure-flow closed curve overlapping the main closed curve during the progress of the actual cycle of man-machine activity.

According to other embodiments, the apparatus comprises a display device on which the pressure-flow graph obtained can be displayed, making it available to a specialized operator.

According to an evolutionary variant, the apparatus comprises, or is associated with, an analysis system, periodic or continuous, of the pressure-flow graph obtained, with the emission of information suitable for the direct or indirect adjustment of the assisted breathing machine, that is, also information suitable to analyze the breathing cycle and to signal alarms.

It is also a variant to provide that the analysis system is able to intervene directly, completely, or within fields defined or definable by the supervisor, on the functioning parameters, correlating them to the needs of the patient.

This intervention can be punctual or delayed as desired, in order to allow the system to verify if the anomaly is punctual or continuous, that is, if a respiratory synchrony is occurring or not.

The main closed curve on which the present invention is based, is generated diagrammatically by processing the pressure and flow parameters. It is the presence of (at least) one small closed pressure-flow curve, overlapping the main pressure-flow closed curve, present in the pressure-flow graph relating to a single respiratory act, which identifies the presence of an asynchrony, and possibly the type of asynchrony, and therefore indicates the intervention factors to return the man-machine synchrony.

In accordance with other embodiments, the processing unit can also be configured to compare the area enclosed by the, or by each, secondary closed pressure-flow curve with the area enclosed by the main pressure-flow closed curve, in order to evaluate the characteristics of the asynchrony associated with that specific anomaly.

According to other embodiments, the apparatus can also comprise a signaling unit, configured to generate a signal in the event that the processing unit identifies an asynchrony, or possibly in the event that the ratio between the area of the secondary closed pressure-flow curve and the main pressure-flow closed curve exceeds a certain threshold value.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a schematic view of an apparatus to control the parameters of an assisted breathing machine connected to an assisted breathing machine;
- fig. 2 is a schematic view of an apparatus to control the parameters of an assisted breathing machine connected to an assisted breathing machine during use;
- figs. 3a and 3b respectively show graphs of the flow as a function of the time of a regular breathing cycle and of a breathing cycle in which there are respiratory asynchronies;
- fig. 4a and 4b respectively show graphs of the flow as a function of the pressure in the case of a regular breathing cycle and of a breathing cycle in which there are respiratory asynchronies.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We shall now refer in detail to the various embodiments of the present invention, of which one or more examples are shown in the attached drawings. For example, the characteristics shown or described insomuch as they are part of one embodiment can be adopted on, or in association with, other embodiments to produce another embodiment. It is understood that the present invention shall comprise all such modifications and variants.

Embodiments described here concern an apparatus 10 to control the parameters of an assisted breathing machine, or pulmonary ventilator 20.

Apparatus 10 according to the invention can be used to identify possible respiratory asynchronies of a patient connected to the pulmonary ventilator 20.

In particular, the apparatus 10 according to the invention can be used to rapidly identify respiratory asynchronies that occur in a complete breathing cycle comprising a delivery phase and an expiration phase.

By the term "rapidly" we mean that respiratory asynchronies can be identified a few moments after they have occurred, or possibly even in real time, that is, at the very moment that they are occurring.

The pulmonary ventilator 20 comprises a ventilation device 22 configured to deliver air and allow expiration, and is provided with a management unit 24 configured to regulate the parameters of the air delivered and expired by the ventilation device 22.

Generally, the management unit 24 comprises, or is connected to, a memory unit 26 in which certain values of the air parameters detected can be memorized, which can comprise volume, pressure, and flow that is to be delivered and expired.

Hereafter we will consider that the parameters used are the flow φ, which identifies the quantity of air in the unit of time, generally measured in liters per minute (1/min), the air pressure P during the delivery phase (positive) and expiration phase (negative), generally measured in centimeters of water (cmH₂O, in which 1 cmH₂O is equal to about 98 Pa) and the volume V of total air delivered and expired, generally measured in liters (1).

According to the invention, pairs of detected parameters are used; in particular, the following pairs of detected parameters can be used: flow and pressure, volume and pressure, and volume and flow.

In particular, the apparatus 10 according to the invention, to identify respiratory asynchronies, provides to use pressure and flow as the parameters.

According to some embodiments, the apparatus 10, to identify respiratory asynchronies, provides to use exclusively pressure and flow as the parameters.

If volume is used, the detection will be such as to provide the volume in the units of time into which the complete cycle is subdivided.

In the memory unit 26, the time durations of the intervals of each inspiration and expiration phase can also be set. Furthermore, the management unit 24 can define, for each unit of time provided, the pressure, volume, and flow of air to be delivered and expired according to the parameters memorized and the unit of time.

The data are obtained with one or more detection devices 28. In accordance with some embodiments, the detection devices 28 can comprise a pressure detection device 28a, a volume detection device 28b, and a flow detection device 28c.

In accordance with some embodiments, the pulmonary ventilator 20 can be connected to, or comprise, a display device 29 in which the time trends of the parameters detected can be displayed, in numerical and/or graphical form.

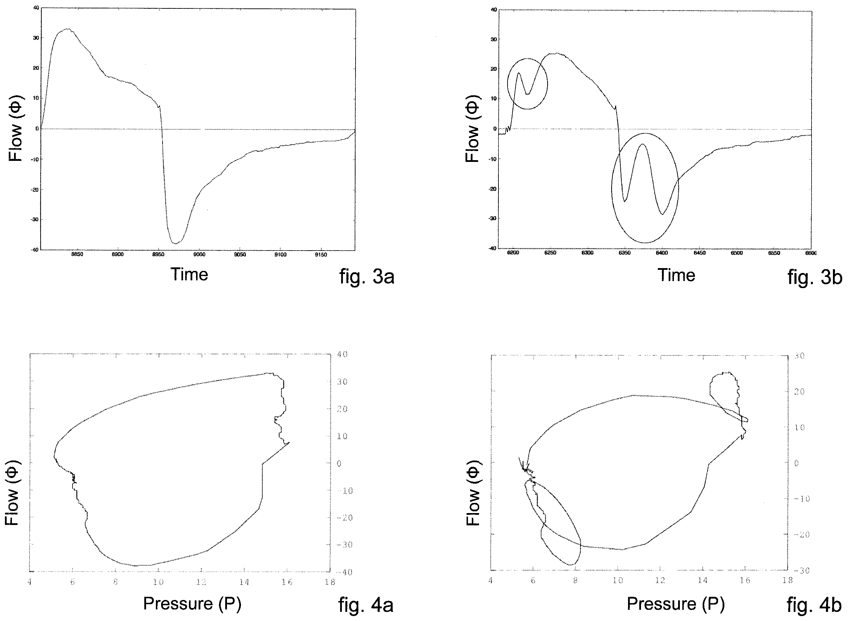

In figs. 3a and 3b, the trend of the flow over time is shown by way of example in a complete breathing cycle, respectively of a regular breathing cycle (fig. 3a) and of a breathing cycle with respiratory asynchronies (fig. 3b). The respiratory asynchronies appear as oscillations that deviate from the temporal trend.

The oscillations in the example shown are obvious, but in practice they can also appear in a form that is not easy to interpret.

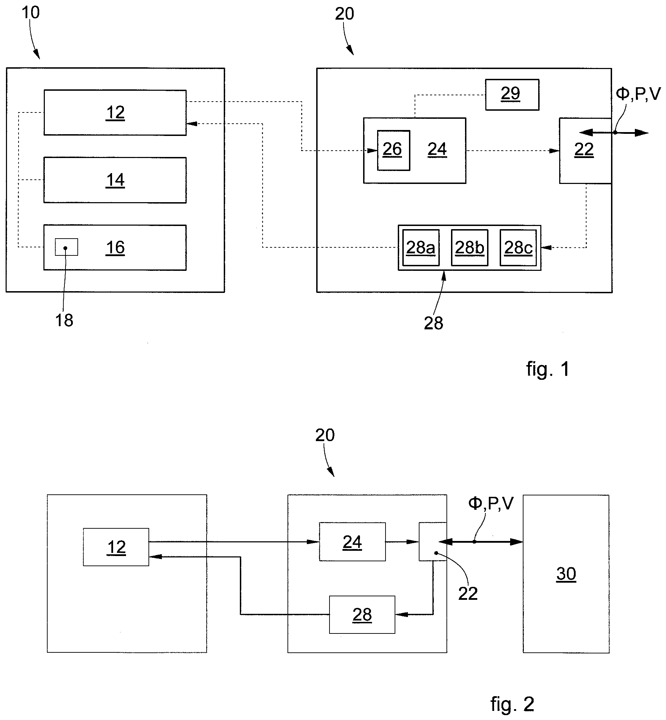

According to some embodiments, for example described with reference to fig. 2, the pulmonary ventilator 20 can be connected to a patient 30, and can be configured to deliver air and allow expiration from the lungs of the patient 30 with a functioning relating to the needs of the patient 30.

In accordance with these embodiments, the data detected by the detection devices 28 can be affected by respiratory activity of the patient 30 himself.

In accordance with one aspect of the present invention, the apparatus 10, during use, is connected to the machine 20 to receive at least the parameters and data detected by the detection devices 28.

The apparatus 10 comprises a processing unit 12 able to receive as input the data detected by the detection devices 28, to select at least two of them in a desired manner, and to process them to identify possible anomalies in the functioning of the pulmonary ventilator 20, corresponding to possible respiratory asynchronies.

In particular, the processing unit 12 can correlate to each other the two parameters selected, and generate a connected plurality of data.

According to a limited solution, the profiles are analyzed by suitable personnel who can possibly intervene on regulating the pulmonary ventilator 20.

According to an evolutionary variant, the data collected by the detection devices 28 can be processed both considering a single pair of parameters correlated to each other among the detected parameters, or as a comparison of two or three pairs of parameters that the detected parameters allow.

In accordance with other variant embodiments, the apparatus 10 can comprise, or be associated with, a processing and analysis system configured to analyze, periodically or continuously, the profile obtained and/or the cycle of delivery/ expiration, and to give out information suitable to regulate the pulmonary ventilator 20.

According to possible solutions, the analysis system can be integrated with or connected to the processing unit 12.

In the case of a single processing, it will be the analysis of the resulting closed circular profile that will be the regulation factor, possibly automatic, of the pulmonary ventilator 20.

In the case of two or three processings, it will be possible for the processing unit 12 to perform all the comparisons necessary before defining the regulation that is most suitable.

The regulation that in one case or another is identified by the processing unit 12 can be translated into instructions for the personnel responsible, or also into instructions for the direct regulation of the pulmonary ventilator 20.

The apparatus 10 can also comprise a display device 14, connected to the processing unit 12, on which the processed data can be displayed, making them available to a specialized operator.

In particular, the processing unit 12 can select the parameters relating to pressure and flow and show them on a pressure-flow graph, so as to construct a main closed curve L1 corresponding to a complete respiratory action, that is, to the breathing cycle comprising the delivery phase and the expiration phase.

In the pressure-flow graph, each point corresponds to the pressure and flow detected at a given instantaneous time.

In the event that a respiratory asynchrony occurs, during the construction of the main closed curve L1 in the pressure-flow graph, a secondary closed curve L2, or minor closed curve, is constructed, overlapping the main pressure-flow curve in relation to a breathing cycle.

The secondary closed curve L2 in particular starts and ends from points of the main closed curve L1 and is substantially a deviation from the profile of the latter.

The secondary closed curve L2 is smaller than the main closed curve L1, that is, the area enclosed by the secondary closed curve L2 is smaller than the area enclosed by the main closed curve L1.

According to some embodiments, in the pressure-flow graph there can also be two or more secondary closed curves L2, L2', both overlapping the same main closed curve L1.

The pressure-flow graph with the respective main closed curve L1 and the possible one or more secondary closed curves L2, L2' can be made visible to a specialized operator on the display device 14.

In figs. 4a and 4b, by way of example, the trend of the flow is shown as a function of the pressure in the case of a regular breathing cycle and a breathing cycle in which there are respiratory asynchronies. From the drawings it is clear that in the case of a normal breathing cycle, the profile in the pressure-flow graph has the shape of a main closed curve L1, generally not uniform but substantially regular.

In the case where there are respiratory asynchronies in the breathing cycle, in the pressure-flow graph there is at least one secondary closed curve L2 or L2', smaller and overlapping the main closed curve L1, where the position in which the overlap occurs depends on the type of asynchrony detected.

Respiratory asynchronies can therefore be identified on the pressure-flow graph as secondary closed curves L2, L2' that define a deviation from the profile of the main closed curve L1, thus at least partly deviating from it.

Depending on the position and/or shape of the anomalies in the resulting pressure-flow chart, it is possible to determine both when they occur, that is, whether in the inspiration or expiration phase, and also the type of anomaly, as well as, possibly, the intensity.

According to other embodiments, the apparatus 10 can also comprise a signaling unit 16 configured to generate a signal if the processing unit 12 identifies an asynchrony, or possibly if the asynchrony is such that a certain safety threshold value is exceeded. The threshold can be fixed on the basis of the ratio between the area of the secondary closed curve L2, L2' and the area of the main closed curve L1.

In accordance with some embodiments, the signaling unit 16 can comprise alarm devices 18 of the acoustic and/or visual type, or of any other type, which intervene if a safety threshold is exceeded.

In accordance with other embodiments, the processing unit 12 can be configured to define the frequency with which possible respiratory asynchronies occur and/or the clinical records.

According to some embodiments, the processing unit 12 can analyze the data relating to a plurality of breathing cycles over a period of time, identify for each individual breathing cycle the possible secondary closed curves L2, L2' overlapping the main closed curve L1, and count them, to calculate the frequency with which the asynchronies occur in said time period.

In accordance with some embodiments, the processing unit 12 can be configured to provide information suitable to intervene on one or more parameters so that the delivery/expiration cycle of the ventilation device 22 is correlated with the needs of the patient 30.

This information can be provided to specialized personnel, for example through the display device 14, or by other suitable means.

In accordance with other embodiments, the processing unit 12 can be configured to directly regulate the functioning of the pulmonary ventilator 20 depending on the asynchronies it detects. For example, the processing unit 12 can be connected to the management unit 24 of the pulmonary ventilator 20, to condition its functioning.

In accordance with some embodiments, the processing unit 12 can define the functioning parameters of the ventilation device 22 and set them by means of the management unit 24 in such a way as to restore the man-machine synchrony.

According to the invention, the regulation deemed appropriate can be provided to the processing unit 12 by the specialized personnel.

According to a variant, the processing unit 12 can be the self-learning type.

In accordance with the above purposes, a method to identify asynchronies in an assisted breathing machine provides to:
- detect data relating to parameters of the pressure, volume and flow of the air delivered and expired by the machine as a function of the time;
- select the pressure-flow pair in order to produce in a pressure-flow graph a main closed curve L1 corresponding to a complete breathing cycle, and a possible secondary closed curve L2 overlapping it in the event that an asynchrony occurs.

According to other embodiments, depending on the deviations from the trend of the profile of the main closed curve L1, defined by one or more secondary closed curves L2, L2', respectively generated by one or more asynchronies, the type and/or the intensity of the corresponding asynchronies, and/or any regulations to be made, are evaluated.

In accordance with possible solutions, depending on the shape and/or type and/or position of the secondary closed curve L2, L2' with respect to the main closed curve L1 that is realized in the pressure-flow diagram, it is possible to identify the type of asynchrony.

According to possible embodiments, the method provides to determine the intensity of a respiratory asynchrony identified. In particular, the intensity can be determined by calculating the ratio between the area enclosed by the secondary curve/curves L2, L2' and the area enclosed by the main closed curve L1 in the pressure-flow graph.

The higher the ratio, the greater will be the intensity of the asynchrony detected. The possibility of quantifying the intensity of an asynchrony in a fast and automatic way, possibly also visually by an operator, allows better monitoring of the respiratory conditions of a patient, and allows to intervene very quickly to restore optimal conditions of assisted breathing.

In accordance with some embodiments, the method can provide to generate an alarm signal to indicate the presence of an asynchrony and/or a significant asynchrony, if the ratio between the areas exceeds a given threshold value.

For example, according to possible embodiments, the method can provide to obtain statistical information on the anomalies, as well as on the modifications of one or the other parameter.

For example, the method can provide to chart the profile of parameters correlated to each other in relation to time, defining a helical curve inside a defined time interval, and to count the anomalies that occur in the trend of the helical curve inside the defined interval to evaluate their frequency.

It is very important to evaluate the frequency with which asynchronies occur, as it has been shown that if the percentage frequency is greater than about 10%, there is a significant increase in the danger of death for the patient 30 assisted.

In accordance with other embodiments, the method can provide to supply information to the specialized personnel regarding the regulation to be made on the machine.

In accordance with other embodiments, the method can provide to directly regulate the functioning of the machine on the basis of the regulation derived from analyzing the profile.

It is clear that modifications and/or additions of parts can be made to the apparatus and method to control the parameters of an assisted breathing machine as described heretofore, without departing from the field and scope of the present invention.

It is also clear that, although the present invention has been described with reference to some specific examples, a person of skill in the art shall certainly be able to achieve many other equivalent forms of apparatus and method to control the parameters of an assisted breathing machine, having the characteristics as set forth in the claims and hence all coming within the field of protection defined thereby.

## Claims

1. Apparatus to identify, in an assisted breathing machine (20), possible respiratory asynchronies between the conditions of inspiration and expiration set by said assisted breathing machine (20) and the activity of inspiration and expiration of a subject associated with said assisted breathing machine (20), said apparatus comprising detection devices (28) configured to detect data relating at least to flow and pressure parameters of the air supplied and expired from said assisted breathing machine (20) and **characterized in that** said apparatus comprises a processing unit (12), connected to said detection devices (28) and configured to select the flow and pressure parameters detected, correlating said flow and pressure parameters with respect to each other to provide said flow and pressure parameters in a pressure-flow graph, constructing at least a main closed pressure-flow curve (L1) that defines a complete breathing cycle, and one or more secondary closed curves (L2, L2') overlapping said main closed curve (L1) in said complete breathing cycle, said one or more secondary closed curves (L2, L2') having respective closed areas below the area enclosed by said main closed curve (L1);
**and in that** said processing unit (12) is configured to analyze the pressure-flow graph obtained in order to identify possible respiratory asynchronies as corresponding to one or more of said secondary closed curves (L2, L2') at least partly deviating from the profile of the main closed pressure-flow curve (L1).

2. Apparatus as in claim 1, **characterized in that** said processing unit (12) provides to use exclusively the pressure and flow parameters.

3. Apparatus as in claim 1 or 2, **characterized in that** it comprises a display device (14), configured to display said pressure-flow graph showing said main closed curve (11) and said one or more secondary closed curves (L2, L2').

4. Apparatus as in any claim hereinbefore, **characterized in that** said processing unit (12) is configured to compare the area enclosed by one or by each secondary closed curve (L2, L2') with the area enclosed by said main closed curve (L1) in the pressure-flow graph in order to determine an intensity of the respiratory asynchrony corresponding to the, or to each, secondary closed curve (L2, L2'), said intensity being determined by calculating a ratio between the area enclosed by said one or more secondary closed curves (L2, L2') and the area enclosed by the main closed pressure-flow curve (L1) in the pressure-flow graph.

5. Apparatus as in claim 4, **characterized in that** it comprises a signaling unit (16), configured to generate an alarm signal by means of an alarm device (18), in the event that the processing unit (12) identifies at least one respiratory asynchrony corresponding to a respective secondary closed curve (L2, L2'), whose intensity exceeds a determinate threshold.

6. Apparatus as in any claim hereinbefore, **characterized in that** it comprises a signaling unit (16), configured to generate a warning signal in the event that the processing unit (12) identifies at least one respiratory asynchrony corresponding to a respective secondary closed pressure-flow curve (L2, L2') overlapping said main closed curve (L1) of the complete breathing cycle.

7. Apparatus as in any claim hereinbefore, **characterized in that** said processing unit (12) can be connected to a management unit (24) of said assisted breathing machine (20), in order to autonomously regulate the parameters of flow, pressure and volume of the air supplied and expired by the latter, as a function of the possible asynchronies identified.

8. Apparatus as in any claim hereinbefore, **characterized in that** said processing unit (12) is configured to analyze the shape of the secondary closed curve (12, L2') and/or its position along the profile of said main closed curve (L1) in order to identify the type of the corresponding respiratory asynchrony.

9. Apparatus as in any claim hereinbefore, **characterized in that** said processing unit (12) is configured to analyze the data relating to a plurality of breathing cycles over a period of time, to identify for each single breathing cycle the possible closed secondary curves (L2, L2') overlapping the main closed curve (L1), and to count them, in order to calculate the frequency with which said respiratory asynchronies occur over said period of time.

## Patentansprüche

1. Vorrichtung zur Identifizierung, in einer Atmungsunterstützungsmaschine (20), möglicher Atmungsasynchronitäten zwischen den durch die Atmungsunterstützungsmaschine (20) festgelegten Einatmungs- und Ausatmungsbedingungen und der Einatmungs- und Ausatmungsaktivität eines mit der Atmungsunterstützungsmaschine (20) verbundenen Individuums, wobei die Vorrichtung Detektionsvorrichtungen (28) aufweist, die so konfiguriert sind, dass sie Daten in Bezug auf mindestens Fluss- und Druckparameter der von der Atmungsunterstützungsmaschine (20) zugeführten und expirierten Luft detektiert,
**dadurch gekennzeichnet, dass** die Vorrichtung eine Verarbeitungseinheit (12) umfasst, die mit den Detektionsvorrichtungen (28) verbunden ist und so konfiguriert ist, dass sie die detektierten Fluss- und Druckparameter selektiert, wobei sie die Fluss- und Druckparameter miteinander korreliert, um die Fluss- und Druckparameter in einem Druck-Fluss-Graphen bereitzustellen, mindestens eine primäre geschlossene Druck-Fluss-Kurve (L1) erstellt, welche einen vollständigen Atmungszyklus definiert, und eine oder mehrere sekundäre geschlossene Kurven (L2, L2') erstellt, welche die primäre geschlossene Kurve (L1) in dem vollständigen Atmungszyklus überlagern, wobei die eine oder die mehreren sekundären geschlossenen Kurven (L2, L2') jeweilige geschlossene Flächen unter der Fläche, die durch die primäre geschlossene Kurve (L1) eingeschlossen wird, aufweist/aufweisen;
und dass die Verarbeitungseinheit (12) so konfiguriert ist, dass sie den erhaltenen Druck-Fluss-Graphen analysiert, um mögliche Atmungsasynchronitäten als korrespondierend zu einer oder mehreren der sekundären geschlossenen Kurven (L2, L2'), die zumindest teilweise von dem Profil der primären geschlossenen Druck-Fluss-Kurve (L1) abweichen, zu identifizieren.

2. Vorrichtung gemäß Anspruch 1, **dadurch gekennzeichnet, dass** die Verarbeitungseinheit (12) ausschließlich die Verwendung der Druck- und Flussparameter vorsieht.

3. Vorrichtung gemäß Anspruch 1 oder 2, **dadurch gekennzeichnet, dass** sie eine Darstellungsvorrichtung (14) umfasst, die so konfiguriert ist, dass sie den Druck-Fluss-Graphen, welcher die primäre geschlossene Kurve (L1) und die eine oder die mehreren sekundären geschlossenen Kurven (L2, L2') zeigt, darstellt.

4. Vorrichtung gemäß irgendeinem der vorhergehenden Ansprüche, **dadurch gekennzeichnet, dass** die Verarbeitungseinheit (12) so konfiguriert ist, dass sie die von einer oder jeder sekundären Kurve (L2, L2') eingeschlossene Fläche mit der von der primären geschlossenen Kurve (L1) eingeschlossenen Fläche in dem Druck-Fluss-Graphen vergleicht, um eine Intensität der Atmungsasynchronität, die der oder jeder sekundären Kurve (L2, L2') entspricht, zu bestimmen, wobei die Intensität durch Berechnen eines Verhältnisses zwischen der durch die eine oder die mehreren sekundären Kurven (L2, L2') eingeschlossenen Fläche und der durch die primäre geschlossene Druck-Fluss-Kurve (L1) eingeschlossenen Fläche in dem Druck-Fluss-Graphen bestimmt wird.

5. Vorrichtung gemäß Anspruch 4, **dadurch gekennzeichnet, dass** sie eine Signalgebungseinheit (16) umfasst, die so konfiguriert ist, dass sie ein Alarmsignal mittels einer Alarmvorrichtung (18) in dem Fall bereitstellt, dass die Verarbeitungseinheit (12) mindestens eine Atmungsasynchronität identifiziert, die einer jeweiligen sekundären geschlossenen Kurve (L2, L2') entspricht, deren Intensität eine vorgegebene Schwelle überschreitet.

6. Vorrichtung gemäß irgendeinem der vorhergehenden Ansprüche, **dadurch gekennzeichnet, dass** sie eine Signalgebungseinheit (16) umfasst, die so konfiguriert ist, dass sie ein Warnsignal in dem Fall bereitstellt, dass die Verarbeitungseinheit (12) mindestens eine Atmungsasynchronität identifiziert, die einer jeweiligen sekundären geschlossenen Druck-Fluss-Kurve (L2, L2'), welche die primäre geschlossene Kurve (L1) des vollständigen Atmungszyklus überlagert, entspricht.

7. Vorrichtung gemäß irgendeinem der vorhergehenden Ansprüche, **dadurch gekennzeichnet, dass** die Verarbeitungseinheit (12) mit einer Verwaltungseinheit (24) der Atmungsunterstützungsmaschine (20) verbunden werden kann, um die Parameter des Flusses, Druckes und Volumens der Luft, die von der Maschine zugeführt und expiriert wird, in Abhängigkeit von den möglichen identifizierten Asynchronitäten autonom zu steuern.

8. Vorrichtung gemäß irgendeinem der vorhergehenden Ansprüche, **dadurch gekennzeichnet, dass** die Verarbeitungseinheit (12) so konfiguriert ist, dass sie die Form der sekundären geschlossenen Kurve (L2, L2') und/oder ihre Position entlang des Profils der primären geschlossenen Kurve (L1) analysiert, um den Typus der entsprechenden Atmungsasynchronität zu identifizieren.

9. Vorrichtung gemäß irgendeinem der vorhergehenden Ansprüche, **dadurch gekennzeichnet, dass** die Verarbeitungseinheit (12) so konfiguriert ist, dass sie die Daten in Bezug auf eine Vielzahl an Atmungszyklen über einen Zeitraum analysiert, um für jeden einzelnen Atmungszyklus die möglichen geschlossenen sekundären Kurven (L2, L2'), welche die primäre geschlossene Kurve (L1) überlagern, zu identifizieren, und sie zählt, um die Häufigkeit, mit der die Atmungsasynchronitäten über den Zeitraum auftreten, zu berechnen.

## Revendications

1. Appareil pour identifier, dans une machine pour la respiration assistée (20), d'éventuelles asynchronismes respiratoires entre les conditions d'inspiration et d'expiration définies par ladite machine pour la respiration assistée (20) et l'activité d'inspiration et d'expiration d'un sujet associée à ladite machine pour la respiration assistée (20), ledit appareil comprenant des dispositifs de détection (28) configurés pour détecter des données relatives au moins à des paramètres d'écoulement et de pression de l'air fourni et expiré de ladite machine pour la respiration assistée (20) et **caractérisé par le fait que** ledit appareil comprend une unité de traitement (12), connectée auxdits dispositifs de détection (28) et configurée pour sélectionner les paramètres d'écoulement et de pression détectés, corrélant lesdits paramètres d'écoulement et de pression l'un par rapport à l'autre pour fournir lesdits paramètres d'écoulement et de pression dans un graphe pression-écoulement, construisant au moins une courbe fermée principal pression-écoulement (L1) qui définit un cycle respiratoire complet et une ou plusieurs courbes fermées secondaires (L2, L2') qui se superposent à ladite courbe fermée principale (L1) dans ledit cycle respiratoire complet, lesdites une ou plusieurs courbes fermées secondaires (L2, L2') ayant des respectives zones fermées au-dessous de la zone délimitée par ladite courbe fermée principale (L1) ;
**et par le fait que** ladite unité de traitement (12) est configurée pour analyser le graphe pression-écoulement obtenu afin d'identifier les éventuels asynchronismes respiratoires correspondant à une ou plusieurs desdites courbes fermées secondaires (L2, L2') s'écartant au moins partiellement du profil de la courbe fermée principale pression-écoulement (L1).

2. Appareil selon la revendication 1, **caractérisé par le fait que** ladite unité de traitement (12) prévoit d'utiliser exclusivement les paramètres de pression et d'écoulement.

3. Appareil selon la revendication 1 ou 2, **caractérisé par le fait qu'il** comprend un dispositif d'affichage (14), configuré pour afficher ledit graphe pression-écoulement montrant ladite courbe fermée principale (L1) et lesdites une ou plusieurs courbes fermées secondaires (L2, L2').

4. Appareil selon l'une quelconque des revendications précédentes, **caractérisé par le fait que** ladite unité de traitement (12) est configurée pour comparer la zone délimitée par une ou par chaque courbe fermée secondaire (L2, L2') avec la zone délimitée par ladite courbe fermée principale (L1) dans le graphe pression-écoulement afin de déterminer une intensité de l'asynchronisme respiratoire correspondant à la ou à chaque courbe fermée secondaire (L2, L2'), ladite intensité étant déterminée en calculant un rapport entre la surface délimitée par lesdites une ou plusieurs courbes fermées secondaires (L2, L2') et la zone délimitée par la courbe fermée principale pression-écoulement (L1) dans le graphe pression-écoulement.

5. Appareil selon la revendication 4, **caractérisé par le fait qu'il** comprend une unité de signalisation (16) configurée pour générer un signal d'alarme au moyen d'un dispositif d'alarme (18), dans le cas où l'unité de traitement (12) identifie au moins un asynchronisme respiratoire correspondant à une respective courbe fermée secondaire (L2, L2'), dont l'intensité dépasse un seuil déterminé.

6. Appareil selon l'une quelconque des revendications précédentes, **caractérisé par le fait qu'**il comprend une unité de signalisation (16) configurée pour générer un signal d'avertissement, dans le cas où l'unité de traitement (12) identifie au moins un asynchronisme respiratoire correspondant à une respective courbe fermée secondaire pression-écoulement (L2, L2') qui se superpose à ladite courbe fermée principale (L1) du cycle respiratoire complet.

7. Appareil selon l'une quelconque des revendications précédentes, **caractérisé par le fait que** ladite unité de traitement (12) peut être connectée à une unité de gestion (24) de ladite machine pour la respiration assistée (20), afin de réguler de manière autonome les paramètres d'écoulement, de pression et de volume de l'air fourni et expiré par ce dernier, en fonction des éventuels asynchronismes identifiés.

8. Appareil selon l'une quelconque des revendications précédentes, **caractérisé par le fait que** ladite unité de traitement (12) est configurée pour analyser la forme de la courbe fermée secondaire (12, L2') et/ou sa position le long du profil de ladite courbe fermée principale (L1) afin d'identifier le type d'asynchronisme respiratoire correspondant.

9. Appareil selon l'une quelconque des revendications précédentes, **caractérisé par le fait que** ladite unité de traitement (12) est configurée pour analyser les données relatives à une pluralité de cycles de respiration sur une période de temps, pour identifier pour chaque cycle de respiration individuel les éventuelles courbes secondaires fermées (L2, L2') qui se superposent à la courbe fermée principale (L1) et pour les compter, afin de calculer la fréquence à laquelle lesdits asynchronismes respiratoires se produisent au cours de ladite période de temps.
